# EUROPEAN PATENT APPLICATION

(11) **EP 3 415 916 A1**
(43) Date of publication of application: **19.12.2018**
(21) Application number: 18186428.1
(22) Date of filing: 03.12.2014
(51) Int. Cl.: G01N 33/574, C12Q 1/48

(54) **METHOD FOR PREDICTING ANTITUMOR EFFICACY OF HSP90 INHIBITOR IN CANCER TREATMENT**

(30) Priority: 16.12.2013 JP 2013259134
(62) Divisional of application: 14871790.3
(71) Applicant: Nippon Kayaku Kabushiki Kaisha, Tokyo 100-0005 (JP)
(72) Inventor: FUKUDA, Tsuyoshi, Tokyo 115-8588 (JP); MIYAZAKI, Osamu, Tokyo 115-8588 (JP); MASUDA, Kuniko, Tokyo 115-8588 (JP); OKAMOTO, Kazuya, Tokyo 115-8588 (JP)
(74) Representative: Graf von Stosch Patentanwaltsgesellschaft mbH

(57) **Abstract**

A method for determining sensitivity of a tumor to a HSP90 inhibitor, including:
measuring a phosphorylation level of Akt or a phosphorylation level of ERK in a tissue of the tumor from a patient.

## Description

### Technical Field

The present invention relates to a biological marker and a method for selecting cancer patients for whom a cancer treatment using an anti-tumor agent with a HSP90 inhibitory activity is expected to be effective.

### Background Art

There is a need to develop novel anti-tumor chemotherapies for treating malignant tumor diseases. Recently, there have been found various functional molecules such as growth factors or growth factor receptors involved in cell growth-related signal transduction and proteins involved in signal transduction pathways. Thus, molecular targeting anti-tumor agents targeting the functional molecules have been developed.

Such molecular targeting anti-tumor agents are expected to be therapeutically effective in some patients but not in others. For example, a BCR-ABL/KIT/PDGFR-A inhibitor Imatinib exhibits a very high therapeutic effect in chronic myeloid leukemia patients with BCR/ABL translocation (NPL 1). EGFR inhibitors Gefitinib and Erlotinib exhibit a high therapeutic effect in lung cancer patients with EGFR gene mutation (NPLs 2 and 3). An anti-HER2 antibody Trastuzumab and a HER2/EGFR inhibitor Lapatinib exhibit a high therapeutic effect in patients with HER2 overexpressing breast cancer (NPLs 4 and 5). An ALK inhibitor Crizotinib has a very high therapeutic effect in lung cancer patients with EML4-ALK gene mutation (NPL 6). Therefore, it is said to be very important to find a therapy-related biomarker in advance before the beginning of a clinical trial in order to make a success of development of novel molecular targeting anti-tumor agents and thus to provide novel treatment options for patients for whom the anti-tumor agent is effective.

A biomarker often does not correspond one-to-one with a compound. For example, although Erlotinib is a different compound from Gefitinib, EGFR mutation is used as a biomarker for both of them. In addition, a biomarker for Trastuzumab, which is an antibody, is the same as one for Lapatinib, which is a small molecular compound. That is, the biomarker for the molecular targeting anti-tumor agent does not depend on a structure but on a mechanism of action of the molecular targeting anti-tumor agent.

One of targets of the molecular targeting anti-tumor agents is a heat shock protein (HSP). The HSP is a molecular chaperone present in a cell, and a functional molecule classified into some families (e.g., HSP90, HSP70, HSP60, HSP40, and small HSPs) depending on its molecular weight. The molecular chaperone is a generic name for proteins which transiently forms a complex with a target protein for the purpose of promoting formation of a functional higher order structure of the target protein. That is, the molecular chaperone assists a protein to be folded or assembled and inhibits a protein from aggregating.

Among them, HSP90 is involved in many in vivo high-order functions such as accurate folding, inhibition of aggregation, intercellular transport, and maintenance of quasi-activated state in 100 types or more proteins. The HSP90 acts by a mechanism in which the HSP90 specifically recognizes and binds to an unstably folded protein to thereby form a complex. In particular, various target proteins involved in cancer-related signal transduction (steroid receptors, Raf serine kinases, tyrosine kinases) depends on the HSP90 for its structural organization. The HSP90 is deeply involved in regulation of cell cycle, and oncogenic-, growth-, and survival-signals of cells.

Regulatory functions of many signal molecules are lost in human tumors, and the HSP90 is needed to maintain the functions (NPL 7). A HSP90 inhibitor changes structure of a chaperon complex containing the HSP90 and its target protein to thereby detach the target protein from the complex, the target protein being degraded mainly via an ubiquitin-proteasome system. This decreases an amount of the target protein for the HSP90. Accordingly, signal transduction downstream of the target protein is blocked, and therefore cancer cells are inhibited from proliferating. Thus, an anti-tumor effect is achieved.

In particular, a plurality of genetic abnormalities are accumulated and proteins are mutated in an oncogenesis process. Cancer cells contains many mutant proteins, so that a higher chaperon activity is needed than normal cells containing normal proteins. Therefore, an expression amount of the HSP90 is increased in many cancer cells. The cancer cells express abnormal proteins, and are under a hypoxic and nutrient starvation condition. That is, the cancer cells are under a kind of stressed condition. It is believed this is why the cancer cells are highly dependent on the chaperon activity of the HSP90. Therefore, the cancer cells are expected to have higher sensitivity to the HSP90 inhibitor than the normal cells. Exploratory studies of the HSP90 inhibitor targeting the HSP90 have been conducted and its anti-tumor effect has been verified.

PTL 1 has described 5-(2,4-dihydroxyphenyl)-[1,2,4]triazol-3-one derivatives as the HSP90 inhibitor. This compound has been reported to exhibit an excellent anti-tumor effect in the experiment on animals and thus be a promising anti-tumor agent. PTLs 2 and 3 have reported triazole compounds having a HSP90 inhibitory activity.

As described above, the HSP 90 is a promising target protein for the anti-tumor agent. However, a biomarker related to a treatment with a HSP90 inhibitor has not been established. PTL 4 has reported, as the biomarker for HSP90, at least one activating mutation in KRAS, EGFR, and BRAF genes. PTL 5 has reported, as the biomarker for the HSP90 inhibitor, mutations in ALK, a MAPK pathway, or an EGFR gene. However, detailed examples were limited to relations between mutations in ALK, KRAS, or EGFR genes and a therapeutic effect of the HSP90 inhibitor. Additionally, there was not a high correlation between mutations in the KRAS and EGFR genes and the effect of the HSP90 inhibitor. In PTL 5, there was a correlation between mutations in the ALK and the effect of the HSP90 inhibitor. However, lung cancer patients with ALK gene mutation are only less than 5% of all lung cancer patients.

PTL 5 has also mentioned mutations in Akt and ERK genes as the biomarker for the HSP90 inhibitor. However, as described above, there is no example in PTL 5 from which the correlation between mutations in the Akt and ERK genes and the therapeutic effect of the HSP90 inhibitor is expected. Criteria based on which mutations in Akt and ERK genes or proteins correlating to the therapeutic effect of the HSP90 inhibitor are selected is not specifically mentioned.

Akt is a serine-threonine kinase acting downstream of a growth factor, and transduces signals relating to growth, survival, differentiation, and carbohydrate metabolism of cells. The Akt is activated by phosphorylation with PDK1/2, which is a kinase acting upstream of the Akt, on a cell membrane (NPL 8). Gene amplification and activating mutations of the Akt have been reported in some classes of cancer, and deficiency of PTEN negatively regulating the Akt has been found in many classes of cancer. Therefore, the anti-tumor agent targeting the Akt have been developed.

ERK is the first discovered MAP kinase, and transduces signals relating to cytoskeleton, cell motility, cell cycle, and differentiation. The ERK is activated by phosphorylation with various growth factors or cell-specific stimuli. The activation of the ERK plays an important role in growth, motility, and survival of cancer cells. An activity of ERK has actually been found to be increased in many classes of cancer (NPL 9).

The phosphorylation of the Akt and the ERK is a signal playing an important role for cell survival and growth. However, there has been no finding that the phosphorylation is directly regulated by HSP90.

### Citation List

### Patent Literature

PTL 1: International Publication No. WO2006/095783
PTL 2: International Publication No. WO2009/023211
PTL 3: International Publication No. WO2007/134678
PTL 4: Japanese Patent Application Laid-Open (JP-A) No. 2012-500013
PTL 5: International Publication No. WO2011/060328

### Non-Patent Literature

NPL 1: Activity of a Specific inhibitor of the BCR-ABL tyrosine kinase in the blast crisis of chronic myeloid leukemia and acute lymphoblastic leukemia with the Philadelphia chromosome. The New England Journal of Medicine. 2001, 344: 1038-42.
NPL 2: Activating mutations in the epidermal growth factor receptor underlying responsiveness of non-small-cell lung cancer to gefitinib. The New England Journal of Medicine. 2004, 350: 2129-39.
NPL 3: Erlotinib versus chemotherapy as first-line treatment for patients with advanced EGFR mutation-positive non-small-cell lung cancer (OPTIMAL, CTONG-0802): a multicentre, open-label, randomised, phase 3 study. The Lancet Oncology. 2011, 12: 735-42.
NPL 4: Use of chemotherapy plus a monoclonal antibody against HER2 for metastatic breast cancer that overexpress HER2. The New England Journal of Medicine. 2001, 344: 783-92.
NPL 5: Lapatinib plus capecitabine for HER2-positive advanced breast cancer. The New England Journal of Medicine. 2006, 355: 2733-43.
NPL 6: Anaplastic lymphoma kinase inhibition in non-small-cell lung cancer. The New England Journal of Medicine.2010, 363:1693-703.
NPL 7: Hsp90 inhibitors as novel cancer chemotherapeutic agents. Trends in Molecular Medicine.2002, 8:4 (Suppl.), S55-61.
NPL 8: Roles of the Raf/MEK/ERK and PI3K/PTEN/Akt/mTOR pathways in controlling growth and sensitivity to therapy-implications for cancer and aging. Aging. 2011, 3:192-222.
NPL 9: Targeting the Raf-MEK-ERK mitogen-actiated protein kinase cascade for the treatment of cancer. Oncogene. 2007, 26: 3291-310.

### Summary of Invention

### Technical Problem

An object of the present invention is to provide a method for identifying a group of patients for whom a HSP90 inhibitor is effective.

### Solution to Problem

The present inventors conducted extensive studies to solve the above-described problems and consequently have found that an expression level of phosphorylated Akt (pAkt) or phosphorylated ERK (pERK) in a tumor correlates to an anti-tumor effect of an HSP90 inhibitor, that is, when an expression amount of pAkt or pERK in a certain tumor is determined to be higher than a control level, the tumor can be determined to be highly sensitive to the HSP90 inhibitor. The present invention is summarized as follows.

The present invention relates to a method for determining sensitivity of a tumor to an HSP90 inhibitor, the method including:
detecting pAkt in a tissue of the tumor from a patient; or
detecting pERK in the tissue of the tumor.

The present invention also relates to a method for selecting a patient for whom a treatment with a HSP90 inhibitor is effective, based on an increased phosphorylation level of Akt as compared with to a control level in a tumor or an increased phosphorylation level of ERK as compared with a control level in the tumor.

The present invention also relates to a method for determining sensitivity of a tumor to an HSP90 inhibitor, the method including:
detecting pAkt in a tissue of the tumor from a patient; and
detecting pERK in the tissue of the tumor.

The present invention also relates to a method, including:
determining a treatment with a HSP90 inhibitor to be effective for a patient when a rate of the number of pAkt-positive cells to the total number of tumor cells in a tumor from the patient is about 1.0% or higher; or
determining the treatment with a HSP90 inhibitor to be effective for a patient when a rate of the number of pERK-positive cells to the total number of tumor cells in a tumor from the patient is about 1.0% or higher.

The present invention also relates to a method, including:
detecting phosphorylation of Akt and/or phosphorylation of ERK using any one or more selected from the group consisting of an immunohistochemical analysis (IHC), a Western blot analysis, an ELISA assay, and a mass spectrometry.

The present invention also relates to the above methods, wherein the HSP90 inhibitor is a triazole compound (A) represented by the following General Formula (1): 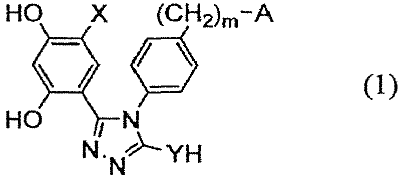 where
X denotes a linear or branched alkyl group having 1 to 8 carbon atoms, an alkynyl group having 2 to 10 carbon atoms, or a halogen atom;
Y denotes a sulfur atom or an oxygen atom;
m denotes an integer of 0 to 4; and
A denotes an amino group having a substituent.

The present invention also relates to the above methods, wherein the HSP90 inhibitor is Ganetespib.

The present invention also relates to the above methods, wherein the tumor is selected from the group consisting of ovarian cancer, breast cancer, small cell lung cancer, non-small cell lung cancer, pancreatic cancer, colorectal cancer, head and neck cancer, endometrial cancer, stomach cancer, esophageal cancer, renal cell cancer, prostatic cancer, skin cancer, leukemia, lymphoma, myeloma, brain tumor, and sarcoma.

### Advantageous Effects of Invention

According to the present invention, a cancer patient for whom a treatment with a HSP90 inhibitor is effective can be selected reliably, easily, and conveniently.

### Brief Description of Drawings

FIG. 1 is a graph illustrating an anti-tumor effect of a triazole compound (A-1) in mice transplanted subcutaneously with human ovarian cancer cells SK-OV-3 (Example 1).
FIG. 2 is a graph illustrating an anti-tumor effect of a triazole compound (A-1) in mice transplanted subcutaneously with human ovarian cancer cells TOV-21G (Example 1).
FIG. 3 is a graph illustrating an anti-tumor effect of a triazole compound (A-1) in mice transplanted subcutaneously with human ovarian cancer cells OV-90 (Example 1).
FIG. 4 is a graph illustrating an anti-tumor effect of a triazole compound (A-1) in mice transplanted subcutaneously with human ovarian cancer cells OVCAR-3 (Example 1).
FIG. 5 is a graph illustrating an anti-tumor effect of a triazole compound (A-1) in mice transplanted subcutaneously with human ovarian cancer cells OC-11-JCK (Example 1).
FIG. 6 is a graph illustrating an anti-tumor effect of a triazole compound (A-1) in mice transplanted subcutaneously with human ovarian cancer cells OC-11-JCK/CDDP (Example 1).
FIG. 7 is a graph illustrating anti-tumor effects of a triazole compound (A-1) and Ganetespib in mice transplanted subcutaneously with human ovarian cancer cells OC-11-JCK/PTX (Example 1).
FIG. 8 is a graph illustrating anti-tumor effects of a triazole compound (A-1) and Ganetespib in mice transplanted subcutaneously with human ovarian cancer cells TOGY-18 (Example 1).
FIG. 9 is a graph illustrating an anti-tumor effect of a triazole compound (A-1) in mice transplanted subcutaneously with human ovarian cancer cells 2008 (Example 1).
FIG. 10 is a graph illustrating an anti-tumor effect of a triazole compound (A-1) in mice transplanted subcutaneously with human ovarian cancer cells SHIN-3 (Example 1).
FIG. 11 is a graph illustrating anti-tumor effects of a triazole compound (A-1) and Ganetespib in mice transplanted subcutaneously with human breast cancer cells MC-19-JCK (Example 1).
FIG. 12 is a graph illustrating anti-tumor effects of a triazole compound (A-1) and Ganetespib in mice transplanted subcutaneously with human breast cancer cells JIMT-1 (Example 1).
FIG. 13 is a graph illustrating anti-tumor effects of a triazole compound (A-1) and Ganetespib in mice transplanted subcutaneously with human lung cancer cells NCI-H1975 (Example 1).
FIG. 14 is a photograph illustrating immunoblot analysis results of proteins EGFR, HER2, HER3, Met, IGF-1R, Raf-1, Stat3, phosphorylated Akt (pAkt), Akt, phosphorylated ERK (pERK), ERK, mTOR, MEK, Hsp90, Hsp70, and Actin in tumors from human ovarian cancer cells OC-11-JCK, OC-11-JCK/CDDP, OC-11-JCK/PTX, ES-2, SHIN-3, SK-OV-3, OVCAR-3, TOV-21G, OV-90, TOGY-18, OC-10-JCK, and 2008 transplanted subcutaneously to mice (Example 1).
FIG. 15 is a photograph illustrating immunoblot analysis results of HSP90-related proteins in a tumor derived from SK-OV-3 sampled before and 4 and 24 hours after administration of a triazole compound (A-1) to mice transplanted subcutaneously with human ovarian cancer cells SK-OV-3 (Example 2).
FIG. 16 is a photograph illustrating immunoblot analysis results of HSP90-related proteins in a tumor derived from TOV-21G sampled before and 4 and 24 hours after administration of a triazole compound (A-1) to mice transplanted subcutaneously with human ovarian cancer cells TOV-21G (Example 2).
FIG. 17 is a graph illustrating rates of the numbers of pAkt-positive cells when tumors derived from human ovarian cancer cells 2008, OO11-JCK, SK-OV-3, and TOV-21G transplanted subcutaneously to mice were immunohistologically stained with anti-pAkt antibodies (Example 3).
FIG. 18 is a graph illustrating rates of the numbers of pERK-positive cells when tumors derived from human ovarian cancer cells OC-11-JCK, SK-OV-3, and TOV-21G transplanted subcutaneously to mice were immunohistologically stained with anti-pERK antibodies.

### Description of Embodiments

As used herein, Akt refers to any or all of Akt1, Akt2, and Akt3, and phosphorylation of Akt (phosphorylated Akt: pAkt) refers to addition of a phosphate group to any amino acid residue in Akt or Akt containing an amino acid residue to which a phosphate group has been added. Specific examples of an amino acid residue to which a phosphate group is to be added include, but not limited thereto, Ser308 or Ser473 (for Akt1), Ser309 or Ser474 (for Akt2), and Ser305 or Ser472 (for Akt3).

As used herein, ERK refers to one or both of ERK1 and ERK2, and phosphorylation of ERK (phosphorylated: pERK) refers to addition of a phosphate group to any amino acid residue in ERK or ERK containing an amino acid residue to which a phosphate group has been added. Specific examples of an amino acid residue to which a phosphate group is to be added include, but not limited thereto, Thr160, Thr177, Thr202, and Tyr204.

As used herein, a phosphorylation level refers to, for a certain protein in a cell, an amount or a rate of a protein containing an amino acid residue to which a phosphate group has been added. For example, a phosphorylation level of Akt refers to an amount of pAkt or a rate of pAkt relative to the total amount of Akt (amount of pAkt + amount of non-phosphorylated Akt). Proteins involved in intracellular signal transduction including Akt and ERK are activated mainly by being phosphorylated. Therefore, it can be presumed that a signal transduction protein having a high phosphorylation level is activated while a signal transduction protein having a low phosphorylation level is not activated.

As used herein, detecting a phosphorylation level of Akt or ERK refers to all techniques for detecting the phosphorylation level of Akt or ERK as described above. Examples of a method for detecting a phosphorylation level of Akt or ERK include immunological detection methods using an anti-phosphorylated Akt antibody or anti-phosphorylated ERK antibody such as a Western blot analysis, an ELISA assay, an immunostaining method, and an immunohistochemical analysis (IHC); methods for detecting pAkt or pERK using a mass spectrometry; detection methods using a functional molecule which specifically interacts with a phosphate group; and detection methods using a functional molecule, a protein, or RNA which specifically interacts with pAkt or pERK.

As used herein, an immunohistochemical analysis (IHC) refers to a common technique for verifying expression of a certain protein in a freezed sample or a paraffin-embedded sample prepared from a specimen.

As used herein, a Western blot analysis refers to a common technique including separating proteins by electrophoresis, transferring the proteins onto a membrane, and then detecting only the intended protein using an antibody.

As used herein, an ELISA assay refers to a detection technique using an antibody specific to the intended protein or phosphorylated residues in combination with an enzymatic reaction. The ELISA assay enables to measure an amount of only a certain substance recognized by the antibody in a specimen including a plurality of substances.

As used herein, a mass spectrometry refers to a technique used for determining a mass-to-charge ratio of a molecule or an ion. The mass spectrometry includes ionizing a sample in a vacuum with a high voltage being applied to thereby electrically or magnetically separate the sample. The sample is separated depending on the mass-to-charge ratio, the mass-to-charge ratio being unique to a substance from which the sample is derived. Thus, the intended substance can be detected. A method for ionizing is not particularly limited herein, but may be MALDI which allows a protein as an ion source to stably ionize.

As used herein, a HSP90 inhibitor refers to all those having an HSP90-inhibiting activity. Examples thereof include anti-HSP90 antibodies, triazole ring-containing compounds, 17-AAG, Geldanamycin, Radicicol, PU3, or derivatives thereof or pharmacologically acceptable salts thereof, all of which have the HSP90-inhibiting activity. Examples of the triazole ring-containing compounds, as one example of the HSP90 inhibitor, include Ganetespib, a triazole compound (A) represented by the following General Formula (1): 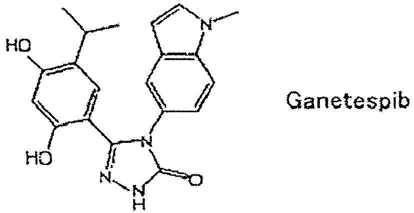 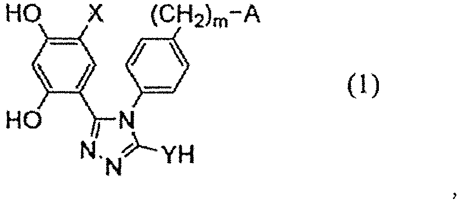 and pharmacologically acceptable salts thereof. Tautomers of Ganetespib and the triazole compound (A) are also included.

In the General Formula (1), X denotes a linear, branched, or cyclic alkyl group having 1 to 20 carbon atoms, an alkynyl group having 2 to 10 carbon atoms, or a halogen atom. When X is the alkyl group, X is preferably a linear or branched alkyl group having 1 to 8 carbon atoms, further preferably an ethyl group, an isopropyl group, a tert-butyl group, or a 2,2-dimethylpropyl group. When X is the alkynyl group, X is preferably an alkynyl group having 2 to 6 carbon atoms, further preferably a 2-propynyl group or a 2-butynyl group. When X is the halogen atom, X is preferably a bromide atom or a chlorine atom.

In the General Formula (1), Y is a sulfur atom or an oxygen atom.

In the General Formula (1), m is an integer of 0 to 4, preferably 0 or 1.

In the General Formula (1), A is not limited so long as it is an amino group having a substituent, but is preferably a morpholino group, a 4-methylpiperazin-1-yl group, a piperidin-1-yl group, or a pyrrolidin-1-yl group.

As used with respect to Ganetespib and the General Formula (1), the tautomers refer to the same compounds as each other which are rapidly interconvertible with each other and in which triazole rings are in the relationship to each other as illustrated in the following General Formulae (2-1) and (2-2):

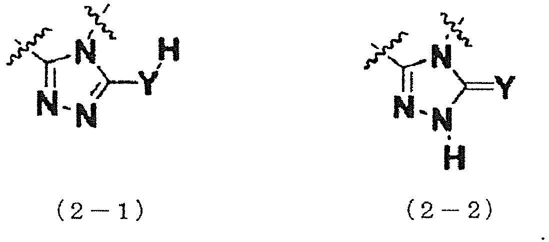

### Examples

The present examples exemplify certain embodiments of the present invention and various use thereof. The present examples are for exemplary purposes only and the present invention is not limited thereto.

Mice (all were female) used in the present examples were as follows:

### Nude mice

Strain: BALB/cA-nu/nu (6 to 13 week-old at transplant)
Supplier: CLEA Japan, Inc. or CHARLES RIVER LABORATORIES JAPAN, INC.

### SCID mice

Strain: CB-17/scid (5 to 7 week-old at transplant)
Supplier: CHARLES RIVER LABORATORIES JAPAN, INC.

### NOD/SCID

Strain: NOD-scid (8 week-old at transplant)
Supplier: CHARLES RIVER LABORATORIES JAPAN, INC.

### Synthetic Example 1: Synthesis of HSP90 inhibitor compound

As the triazole compound (A) represented by the General Formula (1) of the present invention,
5-(2,4-dihydroxy-5-isopropyl-phenyl)-4-[4-(4-methyl-piperazin-1-ylmethyl)-phenyl]-2 ,4-dihydro-[1,2,4]triazol-3-one; (A-1) was used. The (A-1) was synthesized according to the production method disclosed in Example 2-5 of International Publication No. WO2006/095783. Ganetespib was synthesized according to the production method disclosed in Example 4 of International Publication No. WO2007/139952.

### Test Example 1: Anti-tumor test in mice transplanted subcutaneously with a tumor

### <Cells and tumors to be transplanted>

Human ovarian cancer cell lines OC-10-JCK, OC-11-JCK, and TOGY-18, and a human breast cancer cell line MC-19-JCK were obtained from Central Institute for Experimental Animals. Cryopreserved tumor pieces were thawed, and then OC-10-JCK, OC-11-JCK, and MC-19-JCK were transplanted subcutaneously to SCID mice, while TOGY-18 was transplanted subcutaneously to nude mice. After the tumors were grown, the tumors were removed and cut into tumor pieces. The tumor pieces were transplanted subcutaneously to SCID mice or nude mice with trocars. The tumors were maintained by repeated transplantation in the same manner as described above to prepare tumors to be transplanted. Human ovarian cancer cell lines ES-2, SK-OV-3, OV-90, and TOV-21G, and a human lung cancer cell line NCI-H1975 were obtained from American Type Culture Collection (ATCC). The SK-OV-3 and NCI-H1975 cell lines were grown in recommended culture media, of which about 1 × 10⁷ cells were suspended into HBSS and then injected subcutaneously to SCID mice. After the cells formed tumors, the tumors were removed and cut into tumor pieces. The tumor pieces were transplanted subcutaneously to SCID mice with trocars. The tumors were maintained by repeated transplantation in the same manner as described above to prepare tumors to be transplanted. The OV-90 cell line was grown in a recommended culture medium, of which about 1 × 10⁶ cells were suspended into HBSS and then injected subcutaneously to SCID mice. After the cells formed a tumor, the tumor was subjected to an anti-tumor test. The TOV-21G cell line was grown in a recommended culture medium, of which about 1 × 10⁷ cells were suspended into HBSS and then injected subcutaneously to SCID mice. After the cells formed a tumor, the tumor was subjected to the anti-tumor test. The ES-2 cell line was grown in a recommended culture medium, suspended into HBSS, and then injected subcutaneously to nude mice. After the cells formed a tumor, the tumor was removed and cut into tumor pieces. The tumor pieces were transplanted subcutaneously to nude mice with a trocar. The tumor was maintained by repeated transplantation in the same manner as described above to prepare a tumor to be transplanted. A human ovarian cancer cell line OVCAR-3 was obtained from Cell Resource Center for Biomedical Research (CRCBR). The cell line was grown in RPMI1640 medium supplemented with 10% FBS, of which about 1 × 10⁷ cells were suspended into HBSS and then injected subcutaneously to NOD/SCID mice. After the cells formed a tumor, the tumor was subjected to the anti-tumor test. A human breast cancer cell line JIMT-1 was obtained from German Collection of Microorganisms and Cell Cultures (DSMZ). The cell line was grown in a recommended culture medium, suspended into HBSS and then injected subcutaneously to SCID mice. After the cells formed a tumor, the tumor was removed and cut into tumor pieces. The tumor pieces were transplanted subcutaneously to SCID mice with a trocar. The tumor was maintained by repeated transplantation in the same manner as described above to prepare a tumor to be transplanted. A human ovarian cancer cell line 2008 was grown in RPMI1640 medium supplemented with 10% FBS, of which about 1 × 10⁷ cells were suspended into HBSS and then injected subcutaneously to SCID mice. After the cells formed a tumor, the tumor was removed and cut into tumor pieces. The tumor pieces were transplanted subcutaneously to SCID mice with a trocar. The tumor was maintained by repeated transplantation in the same manner as described above to prepare a tumor to be transplanted. A human ovarian cancer cell line SHIN-3 was grown in E-MEM medium supplemented with 15% FBS, of which about 1 × 10⁶ cells were suspended into HBSS and then injected subcutaneously to SCID mice. After the cells formed a tumor, the tumor was subjected to the anti-tumor test. OC-11-JCK/PTX and OC-11-JCK/CDDP were established at Pharmaceuticals Research Laboratories of Nippon Kayaku Co., Ltd. OC-11-JCK obtained from Central Institute for Experimental Animals was transplanted to SCID mice or nude mice, to which paclitaxel (PTX) or cisplatin (CDDP) were repeatedly administered. As a result, a PTX-resistant cell line and a CDDP-resistant cell line, which were designated as OC-11-JCK/PTX and OC-11-JCK/CDDP, were obtained.

### <Anti-tumor test in mice transplanted subcutaneously with tumor>

The above-described tumors derived from OC-10-JCK, OC-11-JCK, OC-11-JCK/CDDP, OC-11-JCK/PTX, MC-19-JCK, TOGY-18, SK-OV-3, ES-2, 2008, NCI-H1975, and JIMT-1 maintained by passage in SDID mice or nude mice were transplanted subcutaneously to the dorsums of SCID mice, NOD/SCID mice (for OVCAR-3 only) or nude mice with trocars. The OV-90, TOV-21G, OVCAR-3, and SHIN-3 cell lines were transplanted subcutaneously to mice in the same manner as described above. When the average tumor volume of each tumor reached about 100 mm³ to about 200 mm³, test drugs were started to be administered. Dosage and usage of the drugs were as follows. The triazole (A-1) synthesized as the HSP90 inhibitor compound in Synthetic Example 1 was dissolved in Japanese Pharmacopoeia glucose injection, and then administered into a tail vein at a dose of 40 mg/kg three times at 4 day intervals. For MC-19-JCK, the triazole was administered once at 40 mg/kg. For NCI-H1975 and JIMT-1, the triazole was administered at 40 mg/kg three times at 7 day intervals. Ganetespib was dissolved in dimethylsulfoxide, diluted 10 times with Japanese Pharmacopoeia glucose injection containing 20% of Cremophor (registered trademark) RH40, and then administered into a tail vein at a dose of 125 mg/kg three times at 7 day intervals. For MC-19-JCK, Ganetespib was administered once at 125mg/kg. For NCI-H1975, Ganetespib was administered into a tail vein at 150 mg/kg three times at 7 day intervals. The tumors were measured twice a week from the beginning of the administration to the completion of an observation period. A long length (L) and a short length (W) of each of the tumors were measured with a caliper. Based on the long length and the short length measured, a tumor volume was calculated according to the following expression: L × W² × 1/2. Each of drug administration groups and a non-administration group (control group) each including 3 mice to 5 mice per group were subjected to the anti-tumor test. From the beginning to the 21st day of administration, a relative tumor volume of each of the drug administration groups (T/C (%)) based on a relative tumor volume of the non-administration group as 100 was calculated as an index of an anti-tumor effect according to the following expression: Relative tumor volume of drug administration group / Relative tumor volume of non-administration group × 100. For each tumor, the lowest T/C (%) value from the beginning to the 21st day of administration is presented in Table 1.

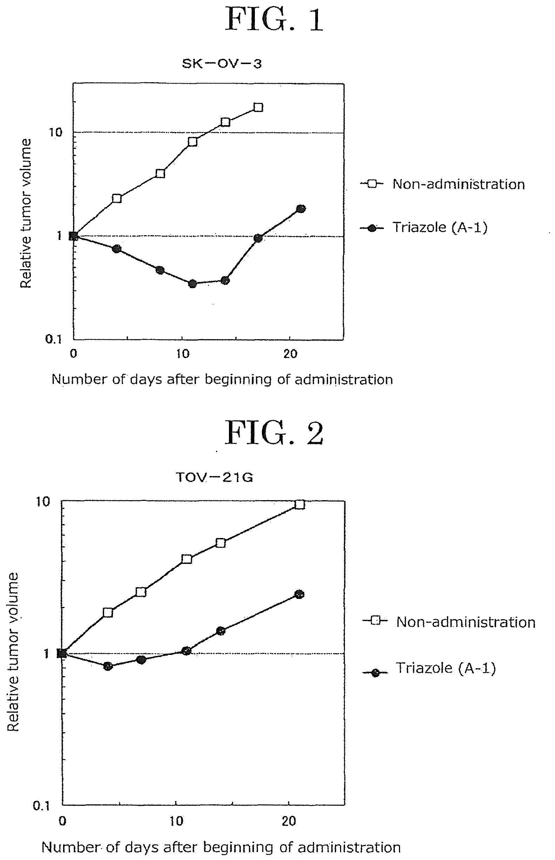

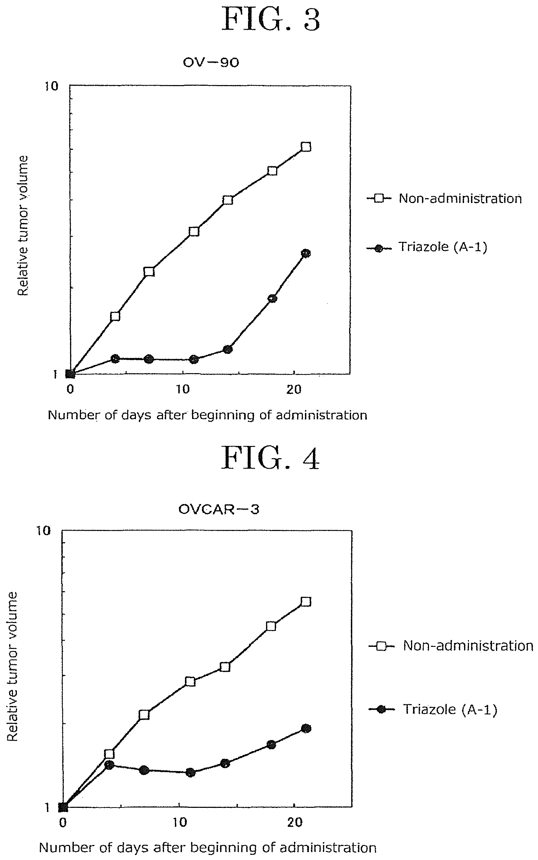

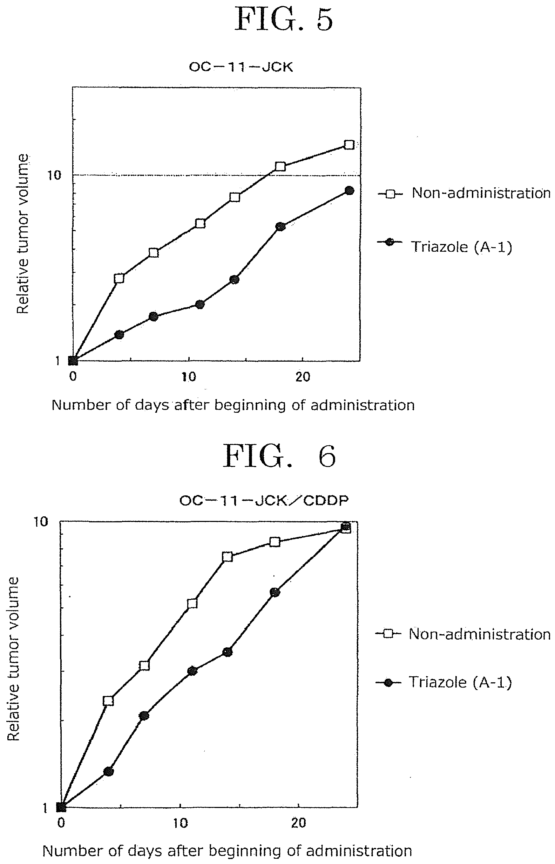

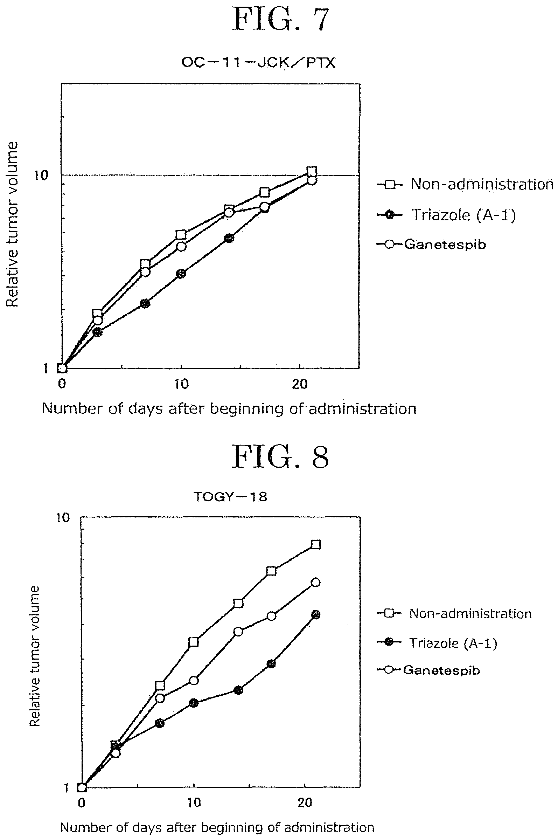

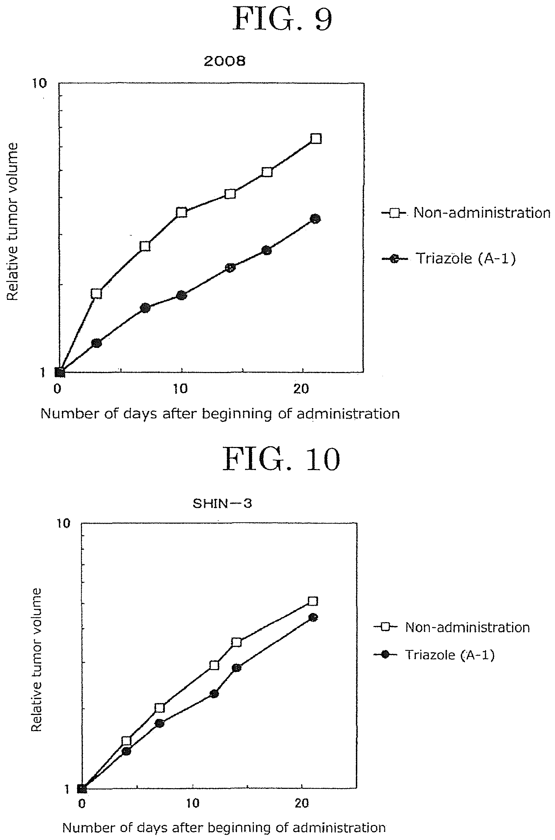

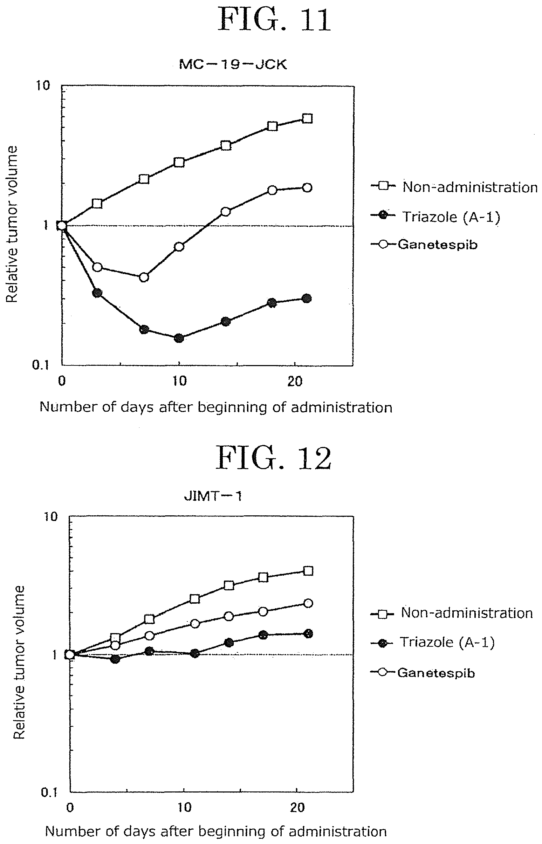

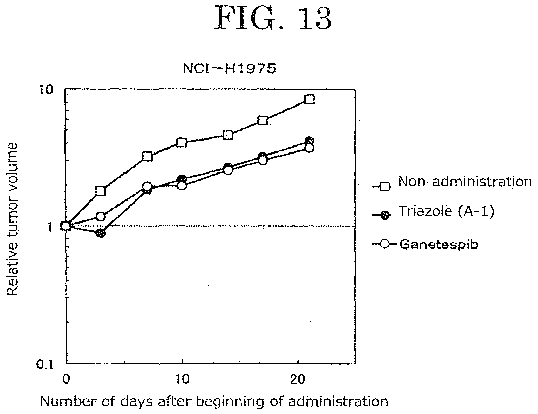

Time-dependent tumor growth curves representing relative tumor volumes based on a tumor volume at the beginning of administration as 1 are illustrated in FIG. 1 (SK-OV-3), FIG. 2 (TOV-21G), FIG. 3 (OV-90), FIG. 4 (OVCAR-3), FIG. 5 (OC-11-JCK), FIG. 6 (OC-11-JCK/CDDP), FIG. 7 (OC-11/PTX), FIG. 8 (TOGY-18), FIG. 9 (2008), FIG. 10 (SHIN-3), FIG. 11 (MC-19-JCK), FIG. 12 (JIMT-1), and FIG. 13 (NCI-H1975).

### <Extraction of tumor protein>

Protein extraction liquid was obtained from each tumor as follows. The human ovarian cancer tumors maintained by passage in SCID mice or nude mice were transplanted subcutaneously to the dorsums of SCID mice or nude mice with trocars. When the tumor volume of each tumor reached about 300 mm³, the tumor was removed, immediately minced, measured for wet weight, and then rapidly freezed with liquid nitrogen. The freezed tumor was thoroughly homogenized with a beads shocker. To this, was added at 4 µL/mg a RIPA buffer containing a protease inhibitor and a phosphatase inhibitor, followed by gently stirring and leaving to stand on ice for 15 min. The resultant cell suspension liquid was centrifuged, of which supernatant was determined as the protein extraction liquid. The total protein amount in the protein extraction liquid was quantified by BioRad protein assay.

### <Expression amount of protein>

An immunoblot method was used to verify expression amounts of target proteins (client proteins) of HSP90 in a tumor and cell survival- and growth-related proteins.

Thirty micrograms of total protein was subjected to electrophoresis in 8% acrylamide gel. Then, the protein was transferred onto a PVDF membrane at a voltage of 100 V for 60 min at 4°C. The PVDF membrane on which the protein had been transferred was blocked in TBS-Tween (0.1%) containing 5% milk at normal temperature for 60 min, followed by incubating with each of primary antibodies described below overnight at 4°C.

### Primary antibody (supplier, product number, dilution ratio, exposure time)

EGFR (Santa Cruz, sc-003, 1:500, 1 min)
HER2 (Santa Cruz, sc-284, 1:2000, 5 sec)
HER3 (Santa Cruz, sc-285, 1:400, 1 min)
Met (Santa Cruz, sc-161, 1:500, 5 min)
IGF-1R (Cell Signaling, #3027, 1:500, 15 sec)
Raf-1 (Santa Cruz, sc-133, 1:300, 5 min)
Stat3 (BD Transduction Laboratory, 610189, 1:500, 15 sec)
pAkt (Cell Signaling, #4058S, 1:500, 1 min)
Akt (Cell Signaling, #4691S, 1:2000, 15 sec)
pERK (Santa Cruz, sc-16982-R, 1:500, 1 min)
ERK (Invitrogen, 44-654G, 1:1000, 15 sec).
mTOR (Cell Signaling, #2983S, 1:500, 15 sec)
MEK (Cell Signaling, #9122S, 1:500, 1 min)
HSP90 (Enzo Life Science, SPA-830, 1:500, 15 sec)
HSP70 (Enzo Life Science, SPA-810, 1:2000, 15 sec)
Actin (Santa Cruz, sc-8432/HRP, 1:300, 5 min)

After incubation, the blot membrane was washed with TBS-Tween (0.1%) at normal temperature for 10 min three times to thereby remove excessive primary antibodies. Then, the membrane was incubated with an appropriate secondary antibody (anti-rabbit IgG HRP conjugate or anti-mouse IgG HRP conjugate) at normal temperature for 60 min. The membrane was washed with TBS~Tween (0.1%) at normal temperature for 10 min three times to thereby remove excessive secondary antibodies. Then, the membrane was incubated with ECL Western Blotting Detection Reagents (GE Healthcare) for 2 min, followed by exposing to an X-ray film. Thus, the signal of the intended protein was detected.

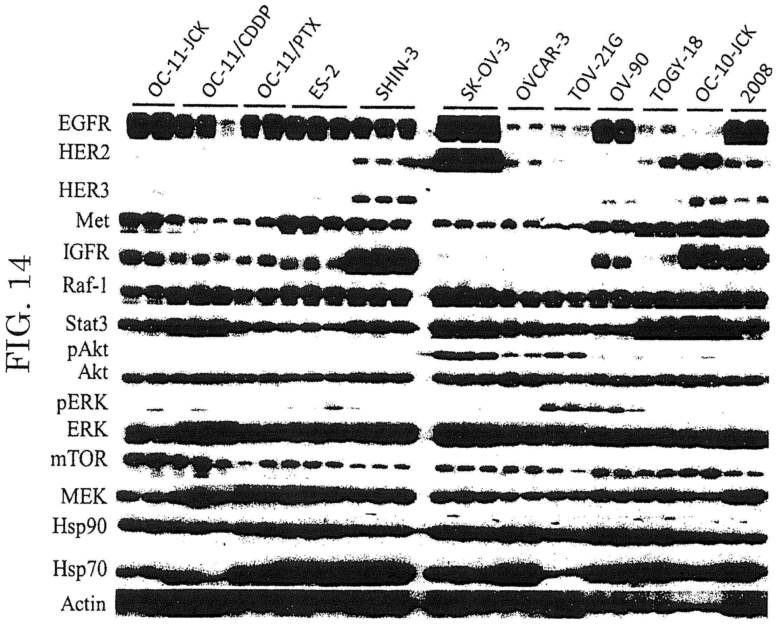

As client proteins, expression amounts of EGFR, HER2, HER3, Met, IGF-1R, Raf-1, Stat-3, Akt, mTOR, and MEK were determined. As cell survival- and growth-regulating proteins, expression amounts of pAkt, pERK, and ERK were determined. As HSP90-related proteins, expression amounts of HSP90 and HSP70 were verified. As an internal control protein, Actin was employed. Protein extraction liquids were collected from at least 2 animals per tumor cell line, and evaluated. Results are presented in FIG. 14. Intensities of expression levels of some proteins are presented in Table 1. The expression levels were determined according to the following criteria:
+++: A very thick clear band was observed at the position corresponding to the intended molecular weight in the Western blot under the above described conditions.
++: A clear band was observed at the position corresponding to the intended molecular weight in the Western blot under the above described conditions.
+: A thin or slightly unclear band was observed at the position corresponding to the intended molecular weight in the Western blot under the above described conditions.
-: No band was observed or only a very thin unclear band was observed at the position corresponding to the intended molecular weight in the Western blot under the above described conditions.

**Table 1: Anti-tumor effects of triazole compound (A-1) and Ganetespib, and expression amounts of various molecular markers**

| Tumor cell line | T/C % | | Expression level | | | | | |
|---|---|---|---|---|---|---|---|---|
| | A-1 | Ganetespib | EGFR | HER2 | Met | pAkt | pERK | mTOR |
| SK-OV-3 | 3.0 | NT | +++ | +++ | + | +++ | - | + |
| TOV-21G | 25.0 | NT | - | - | - | ++ | ++ | - |
| ES-2 | 25.6 | NT | ++ | - | ++ | - | + | + |
| OV-90 | 30.4 | NT | ++ | - | ++ | - | ++ | + |
| OVCAR-3 | 34.9 | NT | - | + | + | + | - | + |
| OC-11-JCK | 36.0 | NT | +++ | - | ++ | - | - | ++ |
| TOGY-18 | 45.2 | 67.7 | - | + | ++ | - | - | + |
| OC-11-JCK/CDDP | 46.4 | NT | + | - | - | - | - | ++ |
| OC-10-JCK | 50.2 | NT | - | ++ | ++ | - | - | ++ |
| 2008 | 51.7 | NT | +++ | + | ++ | - | - | - |
| OC-11-JCK/PTX | 62.4 | 84.0 | ++ | - | + | - | - | + |
| SHIN-3 | 78.1 | NT | + | + | + | - | - | - |
| MC-19-JCK | 5.2 | 19.8 | NT | NT | NT | NT | NT | NT |
| JTMT-1 | 34.9 | 56.6 | NT | NT | NT | NT | NT | NT |
| NCI-H1975 | 49.4 | 44.0 | NT | NT | NT | NT | NT | NT |

| | | | | | | | | |
|---|---|---|---|---|---|---|---|---|
| NT: Not Tested. | | | | | | | | |

It can be seen from Table 1 that, like other molecular target drugs, the triazole compound (A-1) was highly effective for some lines (e.g., SK-OV-3 and TOV-21G) but hardly effective for others (e.g., OC-11-JCE/PTX and SHIN-3).

It can be seen from FIG. 14 or Table 1 that expression amounts of pAkt and pERK in tumor cell lines for which the triazole compound (A-1) was relatively highly effective (SK-OV-3, TOV-21G, ES-2, OV-90, and OVCAR-3) tended to be higher than those in other tumor cell lines, but expression amounts of pAkt and pERK in tumor cell lines for which the triazole compound (A-1) was lowly effective (e.g., 2008, OC-11/PTX, or SHIN-3) tended to be low. Meanwhile, there was not found a correlation between expression amounts of client proteins of HSP90 or HSP90-related proteins and the effect of the triazole compound (A-1).

The above results indicate that the tumor containing an increased expression amount of pAkt or pERK is highly sensitive to the triazole compound (A-1).

It can be seen from Table 1 that there was found a correlation between the anti-tumor effect of the triazole compound (A-1) and the anti-tumor effect of Ganetespib. This is because a biomarker for determining the anti-tumor effect and an expression pattern thereof is common to HSP90 inhibitors containing at least a triazole ring. The biomarker for the molecular targeting anti-tumor agent does not depend on a structure but on a mechanism of action of the molecular targeting anti-tumor agent. Therefore, it is suggested that, in the overall HSP90 inhibitors, the tumor containing an increased expression level of pAkt or pERK is highly sensitive to the HSP90 inhibitor.

### Example 2

### <Cells and tumors to be transplanted>

Tumors to be transplanted derived from human ovarian cancer cell lines SK-OV-3 and TOV-21G were prepared in the same manner as in Example 1.

### <Extraction of tumor protein>

Protein extraction liquid was obtained from each tumor as follows. The human ovarian cancer tumors maintained by passage in SCID mice were transplanted subcutaneously to the dorsums of SCID mice with trocars. When the tumor volume of each tumor reached about 300 mm³, the triazole (A-1) synthesized as the HSP90 inhibitor in Synthetic Example 1 was administered into a tail vein of each of the mice at a dose of 40 mg/kg in the same manner as in Example 1. The tumors were removed before and 4 and 24 hours after administration. At each time point, the tumors were removed from three mice for the SK-OV-3 tumor and two mice for the TOV-21G tumor. Protein extraction liquids were obtained in the same manner as in Example 1. The total protein amount in each of the extraction liquids was quantified in the same manner as in Example 1.

### <Change in expression amount of protein>

The immunoblot method was used to verify change in expression amounts of the client proteins and the HSP90-related proteins in the SK-OV-3 and TOV-21G tumors to which the triazole compound (A-1) had been administered, in the same manner as in Example 1. The types and dilution ratios of primary antibodies and secondary antibodies used were the same as in Example 1.

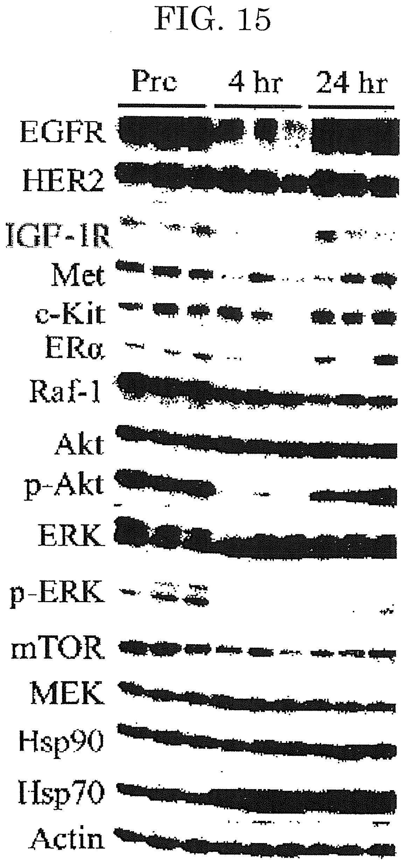

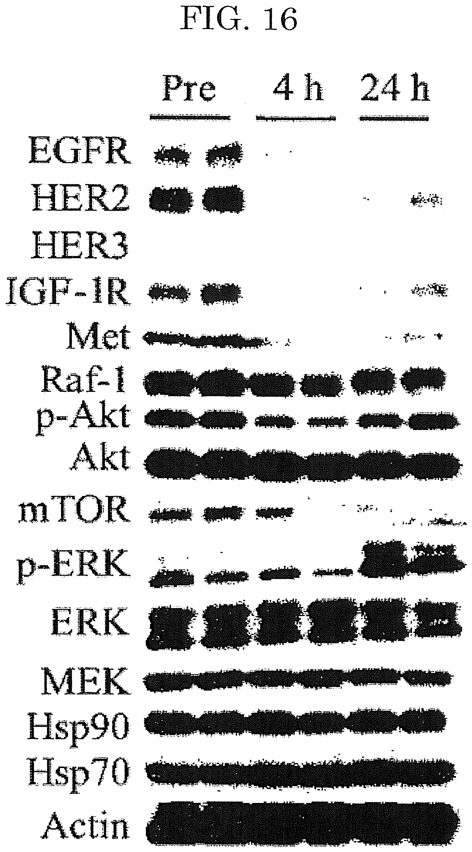

The immunoblot results of the SK-OV-3 tumor are illustrated in FIG. 15 and the immunoblot results of the TOV-21G tumor are illustrated in FIG. 16.

It can be seen from FIG. 15 that expression levels of the client proteins EGFR, IGF-1R, Met, and mTOR in the SK-OV-3 tumor 4 hours after administration of the triazole compound (A-1) were significantly decreased. Accordingly, expression levels of pAkt and pERK, which regulate cell growth- and survival-signals, were also significantly decreased. Meanwhile, expression amounts of total Akt and total ERK were not changed. It can be seen from FIG. 16 that expression levels of the client proteins EGFR, HER2, IGF-1R, Met, and mTOR were also decreased in the TOV-21G tumor. Accordingly, an expression level of pAkt was also decreased. However, the expression amounts of total Akt and total ERK were not changed. The above results indicate that the triazole compound (A-1) serving as the HSP90 inhibitor decreases the expression amounts of the client proteins, which contribute to growth of cancer cells, to thereby inhibit phosphorylation of Akt and ERK, resulting in developing the anti-tumor effect.

### Example 3

### <Cells and tumors to be transplanted>

Tumors to be transplanted derived from human ovarian cancer cell lines SK-OV-3, TOV-21G, 2008, and OC-11-JCK were prepared in the same manner as in Example 1.

### <Production of tissue preparation>

A tissue preparation of each of the tumors was made as follows. The human ovarian cancer tumors maintained by passage in SDID mice were transplanted subcutaneously to the dorsums of SCID mice with trocars. When the tumor volume of each tumor reached about 300 mm³, the tumor was removed. The removed tumor was cut, and immediately thereafter was fixed in 10% neutral buffered formalin overnight. Thus-fixed tumor was dehydrated in ethanol stepwise, cleared in xylene, and then embedded in paraffin. The resultant paraffin block was sectioned at 3 µm and mounted on a glass slide. Thus, a section was produced. The paraffin was removed with xylene from the section. The section was hydrated in ethanol stepwise. After antigens were activated by a microwave treatment in a sodium citrate buffer (this procedure was performed for pAkt staining only), the section was blocked with a 0.2% porcine serum solution at normal temperature for 30 min. Then, the section was incubated with the following primary antibody: pAkt (Sigma, SAB4300259, 1:500) or pERK (Santa Cruz, sc-16982-R, 1:500) overnight at 4°C. The section was washed with TBS buffer at normal temperature for 5 min three times to thereby remove excessive primary antibodies, followed by incubating with anti-rabbit IgG HRP conjugates at 37°C for 30 min. The section was washed with TBS at normal temperature for 5 min three times to thereby remove excessive secondary antibodies. Then, the section was stained with DAB to thereby detect the signal of the intended protein. The stained section of each tumor was observed by a microscope. Photographs were taken in 3 fields of view at an edge of the tumor at which cells were actively grown and which has few necrosis layers. The numbers of positive cells and negative cells among cancer cells were measured and a rate of the positive cells was calculated according to the following equation: Positive cells (%) = 100 × Number of positive cells / (Number of positive cells + Number of non-negative cells).

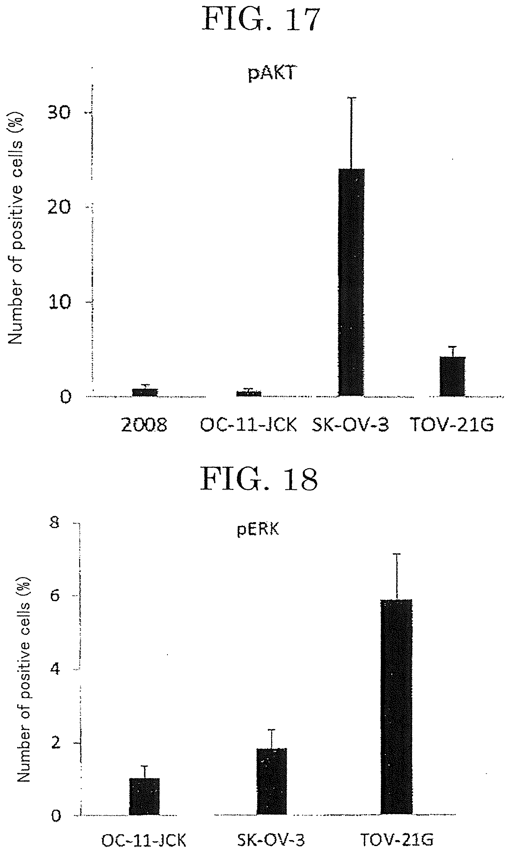

Rates of the pAkt-positive cells in the tumors derived from 2008, OC-11-JCK, SK-OV-3, and TOV-21G are illustrated in FIG. 17. Rates of the pERK-positive cells in the tumors derived from OC-11-JCK, SK-OV-3, and TOV-21G are illustrated in FIG. 18. The rate of each of the positive cells and T/C (%) of the triazole compound (A-1) obtained in Example 1 are presented in Table 2.

**Table 2: Rates of pAkt- and pERK-positive cells in each tumor cell line and anti-tumor effect of triazole compound (A-1)**

| Tumor | pAkt-positive cells (%) | pERK-positive cells (%) | T/C (%) |
|---|---|---|---|
| 2008 | 0.88 | NT | 51.7 |
| OC-11-JCK | 0.67 | 1.01 | 36.0 |
| TOV-21G | 4.31 | 5.89 | 25.0 |
| SK-OV-3 | 24.05 | 1.84 | 3.0 |

It can be confirmed from FIGs. 17 and 18 and Table 2 that there was also a correlation between the rates of pAkt- and pERK-positive cells and the anti-tumor effect of the triazole compound (A-1) in the immunohistological study. From above results, it is suggested that the triazole compound (A-1) is not highly effective for tumors containing less than 1% of both of the pAkt- and pERK-positive cells. The biomarker for the molecular targeting anti-tumor agent does not depend on a structure but on a mechanism of action of the molecular targeting anti-tumor agent. Therefore, it is suggested that, in the overall HSP90 inhibitors, the tumor containing an increased expression level of pAkt or pERK is highly sensitive to the HSP90 inhibitor.

### Items

1. A method for determining sensitivity of a tumor to an HSP90 inhibitor, the method comprising:
   (1) detecting a phosphorylation level of Ait; or
   (2) detecting a phosphorylation level of ERK
   in a tissue of the tumor from a patient.
2. A method for selecting a patient for whom a treatment with a HSP90 inhibitor is effective, the method comprising:
   selecting the patient based on:
   (1) an increased phosphorylation level of Akt as compared with a control level; or
   (2) an increased phosphorylation level of ERK as compared with a control level
   in a tissue of a tumor from the patient.
3. The method according to item 1 or 2, wherein the HSP90 inhibitor is a triazole ring-containing compound.
4. The method according to item 3, wherein the triazole ring-containing compound is Ganetespib.
5. The method according to item 3, wherein the triazole ring-containing compound is a triazole compound (A) represented by the following General Formula (1): 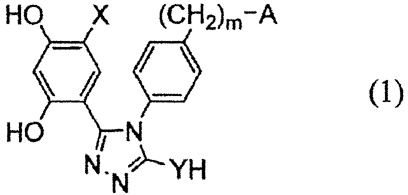 where
   X denotes a linear or branched alkyl group having 1 to 8 carbon atoms, an alkynyl group having 2 to 10 carbon atoms, or a halogen atom;
   Y denotes a sulfur atom or an oxygen atom;
   m denotes an integer of 0 to 4; and
   A denotes an amino group having a substituent.
6. The method according to item 5, wherein X in the General Formula (1) is an ethyl group, an isopropyl group, a tert-butyl group, a 2,2-dimethylpropyl group, a 2-propynyl group, a 2-butynyl group, or a halogen atom.
7. The method according to item 5, wherein, in the General Formula (1), m is 0 or 1 and A is a morpholino group, a 4-methylpiperazin-1-yl group, a piperidin-1-yl group, or a pyrrolidin-1-yl group.
8. The method according to any one of items 1 to 7, wherein at least one of phosphorylation of Akt and phosphorylation of ERK is detected with any one or more selected from the group consisting of an immunohistochemical analysis (IHC), a Western blot analysis, an ELISA assay, and a mass spectrometry.
9. The method according to any one of items 1 to 8, wherein a rate of a number of phosphorylated Akt-positive cells to a total number of tumor cells in the tumor from the patient is 1.0% or higher.
10. The method according to any one of items 1 to 8, wherein a rate of a number of phosphorylated ERK-positive cells to a total number of tumor cells in the tumor from the patient is 1.0% or higher.
11. The method according to any one of items 1 to 10, wherein a cancer is selected from the group consisting of ovarian cancer, breast cancer, small cell lung cancer, non-small cell lung cancer, pancreatic cancer, colorectal cancer, head and neck cancer, endometrial cancer, stomach cancer, esophageal cancer, renal cell cancer, prostatic cancer, skin cancer, leukemia, lymphoma, myeloma, brain tumor, and sarcoma.
12. The method according to any one of items 1 to 10, wherein a cancer is selected from the group consisting of ovarian cancer, breast cancer, small cell lung cancer, and non-small cell lung cancer.
13. The method according to any one of items 1 to 10, wherein a cancer is ovarian cancer.

## Claims

1. A method for determining sensitivity of a tumor to an HSP90 inhibitor, the method comprising:
(1) detecting an increased phosphorylation level of Akt in a tissue of the tumor from a patient,
(2) selecting cancer patients for whom a treatment with a HSP90 inhibitor is expected to be effective by determining the rate of the number of pAkt-positive cells to the total number of tumor cells in the tumor cells from the patient and by selecting patients for whom the rate is 1.0% or higher.

2. The method according to claim 1, wherein the HSP90 inhibitor is a triazole ring-containing compound.

3. The method according to claim 2, wherein the triazole ring-containing compound is Ganetespib.

4. The method according to claim 2, wherein the triazole ring-containing compound is a triazole compound (A) represented by the following General Formula (1): 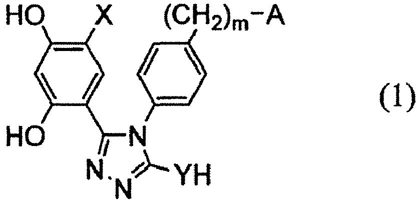 where
X denotes a linear or branched alkyl group having 1 to 8 carbon atoms, an alkynyl group having 2 to 10 carbon atoms, or a halogen atom;
Y denotes a sulfur atom or an oxygen atom;
m denotes an integer of 0 to 4; and
A denotes an amino group having a substituent.

5. The method according to claim 4, wherein X in the General Formula (1) is an ethyl group, an isopropyl group, a tert-butyl group, a 2,2-dimethylpropyl group, a 2-propynyl group, a 2-butynyl group, or a halogen atom.

6. The method according to claim 4, wherein, in the General Formula (1), m is 0 or 1 and A is a morpholino group, a 4-methylpiperazin-1-yl group, a piperidin-1-yl group, or a pyrrolidin-1-yl group.

7. The method according to any one of claims 1 to 6, wherein phosphorylation of Akt is detected with any one or more selected from the group consisting of an immunohistochemical analysis (IHC), a Western blot analysis, an ELISA assay, and a mass spectrometry.

8. The method according to any one of claims 1 to 7, wherein a cancer is selected from the group consisting of ovarian cancer, breast cancer, small cell lung cancer, non-small cell lung cancer, pancreatic cancer, colorectal cancer, head and neck cancer, endometrial cancer, stomach cancer, esophageal cancer, renal cell cancer, prostatic cancer, skin cancer, leukemia, lymphoma, myeloma, brain tumor, and sarcoma.

9. The method according to any one of claims 1 to 7, wherein a cancer is selected from the group consisting of ovarian cancer, breast cancer, small cell lung cancer, and non-small cell lung cancer.

10. The method according to any one of claims 1 to 7, wherein a cancer is ovarian cancer.
